# DEMANDE DE BREVET EUROPEEN

(11) **EP 3 903 835 A1**
(43) Date de publication de la demande: **03.11.2021**
(21) Numéro de dépôt: 21170742.7
(22) Date de dépôt: 27.04.2021
(51) Int. Cl.: A61L 2/06, A61L 2/20, A61L 2/26

(54) **ARMOIRE DE DÉCONTAMINATION PAR L'OZONE, EN PARTICULIER POUR LA DÉGRADATION DES HAP ET/OU LA DESTRUCTION DE MICRO-ORGANISMES**

(30) Priorité: 28.04.2020 FR 2004210
(71) Demandeur: Prodlab, 85000 LA ROCHE-SUR-YON (FR)
(72) Inventeur: KIMMERLING, Olivier, 75010 Paris (FR); GENDRE, Dany, 85190 Aizenay (FR); PIETERS, Vincent, 50610 Jullouville (FR); HALGAND, Laurent, 85600 Montaigu Vendée (FR)
(74) Mandataire: Dupuis-Latour, Dominique

(57) **Abrégé**

L'armoire (10) comprend une enceinte close (30) recevant les effets à décontaminer, des moyens d'admission d'air neuf (78, 80) et une unité de traitement d'air avec un motoventilateur (50), un générateur d'ozone et un élément chauffant pour créer un flux d'air pulsé (APL) chauffé et ozoné à une teneur comprise entre 0,5 et 3 ppm. Une unité d'extraction et de filtrage (62, 64, 66) extrait et filtre une fraction (AEX) de l'air contaminé (ACT). Une paroi de fond ou de plancher (18) de l'armoire comprend des ouïes de diffusion (40) de l'air pulsé (APL) alimentées par un collecteur (76) relié à l'unité de traitement d'air. Le flux d'air pulsé (APL) est diffusé par les ouïes de diffusion (40) essentiellement en partie inférieure (32) de l'enceinte.

## Description

L'invention concerne une armoire de décontamination, en particulier pour la dégradation des hydrocarbures aromatiques polycycliques (HAP) et/ou la destruction de microorganismes présents sur des effets contaminés. Un exemple d'effet contaminé est celui des tenues, bottes, gants, etc. et autres équipements de protection individuelle (EPI) des sapeurs-pompiers après une intervention de lutte contre un incendie : ces effets sont alors contaminés par des produits de réaction d'origine pyrolytique provenant de la combustion incomplète des matières organiques, et majoritairement constituées de HAP toxiques, très stables chimiquement et difficilement dégradables.

L'ozone est reconnu à cet égard comme un réactif à fort pouvoir oxydatif permettant de briser les cycles et les doubles liaisons des HAP pour permettre leur oxydation et leur dégradation en produits de réaction finale non toxiques. Plus précisément, le mécanisme réactionnel majoritaire de l'ozone avec les HAP est un mécanisme dit de Langmuir-Hinshelwood, avec une première phase d'adsorption rapide, sans réaction, de l'ozone à la surface des particules contaminantes, suivie d'une réaction de surface, plus lente, durant laquelle l'ozone implanté à la surface des particules va réagir avec les sites réactionnels en surface, cette dernière réaction étant favorisée et accélérée par une circulation forcée du flux d'air chargé d'ozone. Le taux d'ozone dans l'air nécessaire pour parvenir à la destruction des HAP peut atteindre 2,5 à 3 ppm.

Ce comportement chimique de l'ozone (mécanisme de Langmuir-Hinshelwood) permet également, indépendamment ou en complément, la destruction de microorganismes (bactéries, virus, moisissures, etc.), par lyse des acides gras insaturés, des glycoprotéines, des glycolipides, des acides aminés, de certaines enzymes et finalement de l'ADN du fait du pouvoir oxydatif très élevé de l'ozone, typiquement six fois supérieur à celui du chlore. À la différence de la décontamination chimique par un flux d'air ozoné, ce type de décontamination biologique est habituellement opéré en phase liquide par de l'eau traitée à l'ozone, un traitement par flux d'air étant toutefois également possible. Ici encore, il convient de prévoir des taux d'ozone élevés, typiquement de l'ordre de 1,5 à 2,5 ppm.

Le WO 2019/113434 A1 (Oshkosh Corp.) décrit un véhicule d'intervention pour la lutte contre l'incendie comportant une chambre de décontamination où peuvent être enfermés les effets contaminés des sapeurs-pompiers après l'intervention. Pour la décontamination, cette chambre est balayée par un flux d'air extérieur, avec à une extrémité de la chambre injection d'ozone et d'humidité et, à l'extrémité opposée, aspiration de l'air contaminé au travers d'un filtre avant évacuation vers l'extérieur.

Le US 4 625 432 A (Baltes) décrit une armoire servant à sécher et stériliser un linge déjà lavé, notamment dans le domaine médical (serviettes, blouses, etc.), donc *a priori* propre et non contaminé de polluants. De l'air est brassé avec un flux sensiblement horizontal, et le surplus d'air est évacué par une simple fuite naturelle au travers d'ouvertures ménagées dans le plancher de l'armoire, donc sans maîtrise de la quantité d'ozone rejetée dans l'environnement, à la différence notamment de ce que prévoit le WO 2019/113434 A1 précité.

L'ozone est également souvent proposé dans des applications domestiques de "purification" ou "assainissement" de l'air (élimination des mauvaises odeurs, etc.), mais alors avec des teneurs d'ozone bien plus faibles, typiquement de l'ordre de 0,2 ppm, afin de ne pas engendrer de risques pour l'environnement ni pour la santé des utilisateurs, souvent non professionnels. Le WO 2018/167528 A1 (Bluezone IP Holding LLC) décrit un tel type d'appareil, mettant en œuvre un circuit fermé de recirculation et de filtrage d'un flux d'air brassé chargé d'ozone dans un volume clos où cet air est mis en circulation continue.

Dans le cas de la présente invention, il s'agit d'utiliser des teneurs en ozone bien plus élevées (typiquement cinq à dix fois supérieures à celles des applications de "purification" d'air), donc potentiellement dangereuses si l'air ozoné était rejeté sans précautions particulières dans l'environnement à de telles concentrations.

Un autre facteur à considérer est le fait que les phénomènes décrits plus haut permettant une telle décontamination chimique et/ou biologique par un flux d'air à forte teneur en ozone peuvent être nettement favorisés et accélérés par une élévation de la température de l'air chargé en ozone. On constate en effet que des taux d'ozone à partir de 0,8 ppm combinés à des températures allant jusqu'à 50 °C, voire 60 ou 65 °C si les effets à décontaminer le supportent, il est possible, même pour des effets lourdement chargés en contaminants et polluants divers, d'arriver à une décontamination très efficace en un temps relativement acceptable (typiquement de 2 à 6 heures).

Le chauffage de l'air présente l'avantage additionnel d'accélérer le séchage des effets à décontaminer, qui sont souvent non seulement pollués mais également mouillés, comme dans le cas des équipements des sapeurs-pompiers appelés pour éteindre un incendie.

L'un des buts de l'invention est de proposer une armoire de décontamination à l'ozone incluant des moyens optimisés de brassage d'un flux d'air ozoné chauffé et recirculé en continu dans l'armoire close contenant les effets à traiter. En particulier, il s'agit d'adapter la géométrie et la configuration des différents éléments de l'armoire et de ses équipements pour permettre non seulement une décontamination efficace et rapide, mais également une optimisation du bilan thermique global de l'ensemble (qui doit pouvoir fonctionner plusieurs heures d'affilée avec une consommation énergétique raisonnable), le tout sans rejet dans l'environnement de polluants ni d'air excessivement chargé en ozone.

À cet effet, l'invention propose une armoire du type général divulgué par le US 4 625 432 A précité et correspondant au préambule de la revendication 1, comprenant en outre les éléments spécifiques énoncés par la partie caractérisante de cette revendication 1.

Les sous-revendications visent des formes de réalisation subsidiaires avantageuses.

On va maintenant décrire un exemple non limitatif de mise en œuvre de l'invention en référence aux dessins annexés, où les mêmes références désignent d'une figure à l'autre des éléments identiques ou fonctionnellement semblables.
Figure 1 représente, en perspective, une armoire de décontamination selon un premier mode de réalisation de l'invention.
Figure 2 est une vue de face, en élévation, de l'enceinte de la Figure 1.
Figure 3 est une vue perspective de l'unité de traitement d'air de l'armoire des Figures 1 et 2, avec le capot supérieur retiré pour en montrer les différents éléments constitutifs.
Figure 4 est une vue en coupe transversale de l'unité de traitement d'air de la Figure 3.
Figure 5 montre l'unité de traitement d'air des Figures 3 et 4, vue de dessous en perspective.
Figure 6 est un graphique montrant l'évolution du taux d'ozone à l'intérieur de l'enceinte de l'armoire au cours du temps.
Figure 7 est une vue en coupe transversale de l'armoire des Figures 1 et 2, explicitant la manière dont circulent les différents flux d'air pendant le traitement de décontamination.
Figure 8 est homologue de la Figure 7 pour un second mode de réalisation de l'armoire de l'invention, mettant en œuvre une paroi à double fond formant échangeur thermique.
Figure 9 est une vue agrandie de la partie supérieure arrière de l'armoire illustrée Figure 8.

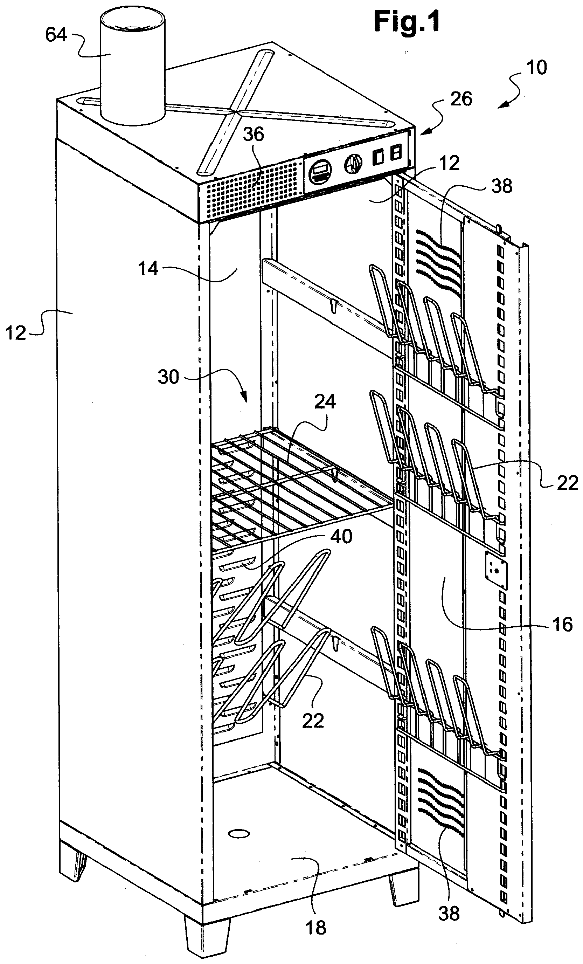

On va d'abord décrire l'agencement d'une armoire de décontamination selon un premier mode de réalisation de l'invention, en référence aux Figures 1 à 5.

L'armoire de décontamination 10 se présente sous forme d'un meuble en hauteur, avec un volume intérieur défini par deux parois verticales de côté 12, une paroi verticale de fond 14, une porte 16, une paroi de plancher 18 et une paroi de plafond 20. Des accessoires divers tels que des supports 22 formant râtelier pour des gants ou des bottes, une grille 24 pour poser des petits objets, une tringle sous la paroi de plafond, etc., permettent de placer dans l'armoire les effets à décontaminer en laissant suffisamment de place autour pour que l'air intérieur puisse circuler autour d'eux.

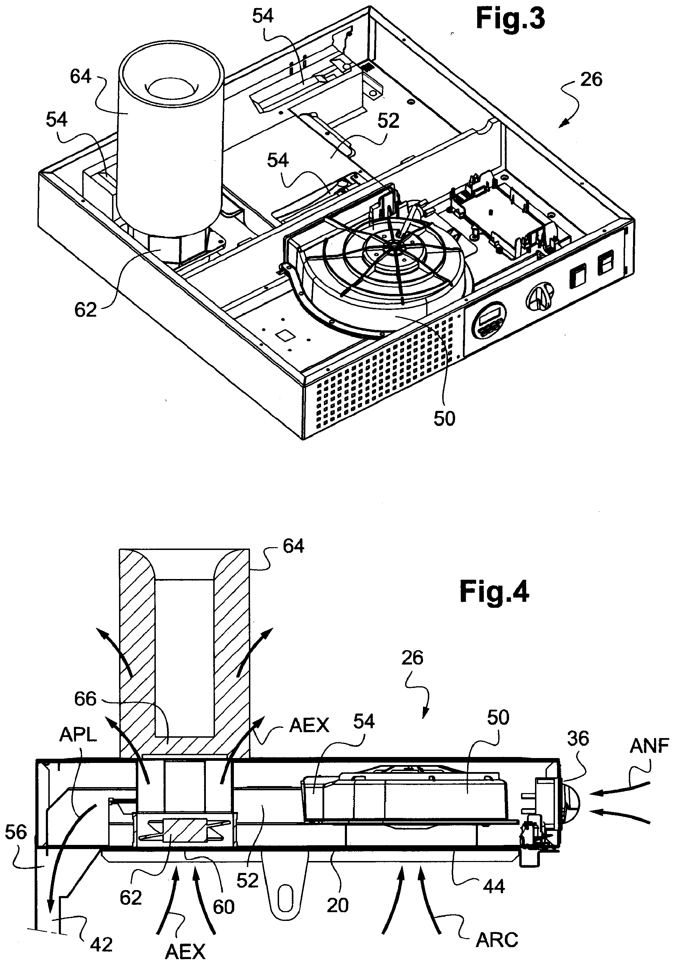

L'armoire est surmontée par un bloc technique 26 intégrant des éléments qui seront décrits plus précisément en référence aux Figures 3 à 5, ainsi que des commandes en façade pour le réglage des paramètres de fonctionnement (température, durée de fonctionnement, etc.) et la surveillance du processus (taux d'ozone, température interne, sécurités diverses). Ce bloc technique est de préférence disposé au sommet plutôt qu'en bas de l'armoire, ce qui le protège notamment des éclaboussures diverses, notamment lors d'un nettoyage à grande eau de l'armoire.

Les parois 12 à 20 de l'armoire définissent un volume intérieur formant une enceinte close 30, à laquelle il est possible d'accéder par ouverture de la porte 16. Cette dernière peut comporter, de manière en elle-même connue, des sécurités de verrouillage empêchant son ouverture tant que la température interne et le taux d'ozone dans l'enceinte ne sont pas descendus au-dessous d'un seuil de sécurité.

L'enceinte 30, qui présente une forme essentiellement verticale (sa hauteur est plus grande que sa profondeur) comporte une partie inférieure 32 et une partie supérieure 34, la partie inférieure correspondant à 30-70 % de la hauteur totale de l'enceinte, de préférence 40-60 %, par exemple 50 % comme dans l'exemple illustré sur les figures.

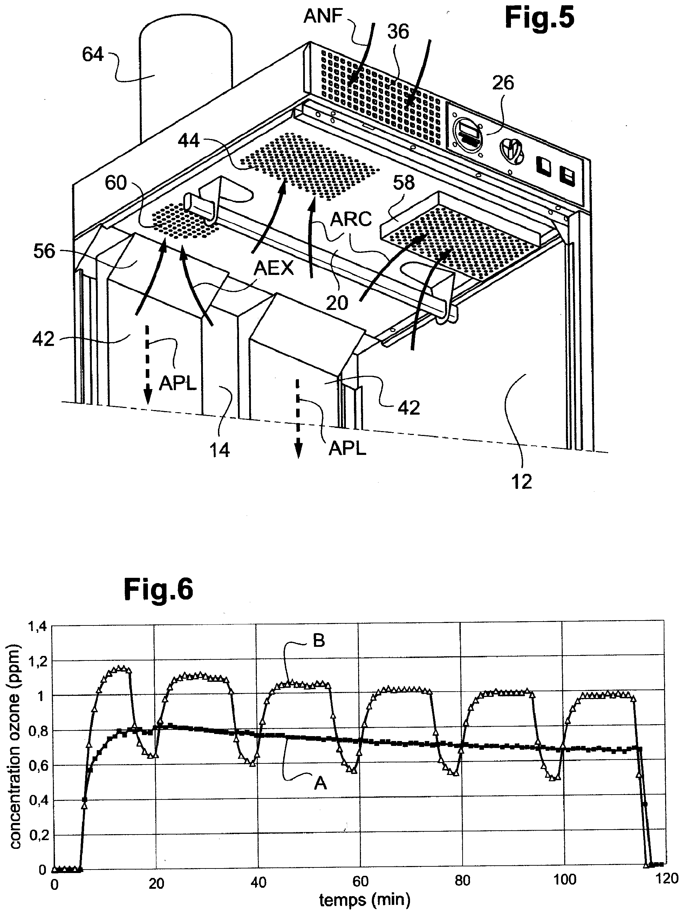

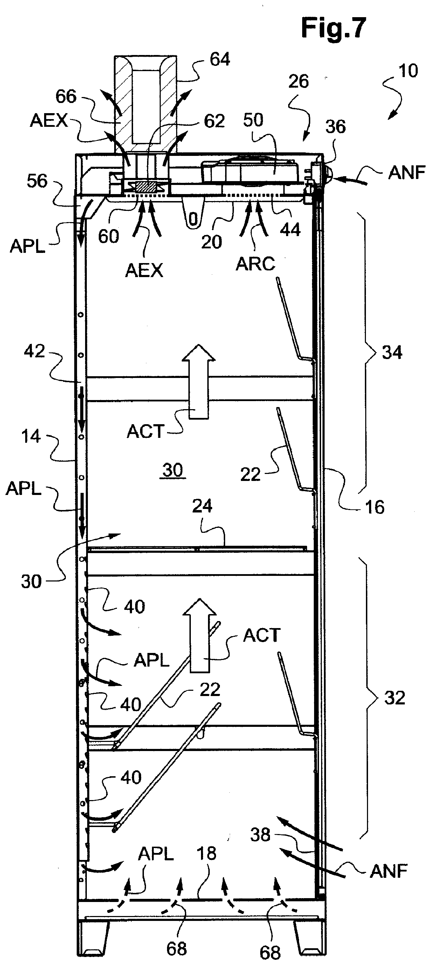

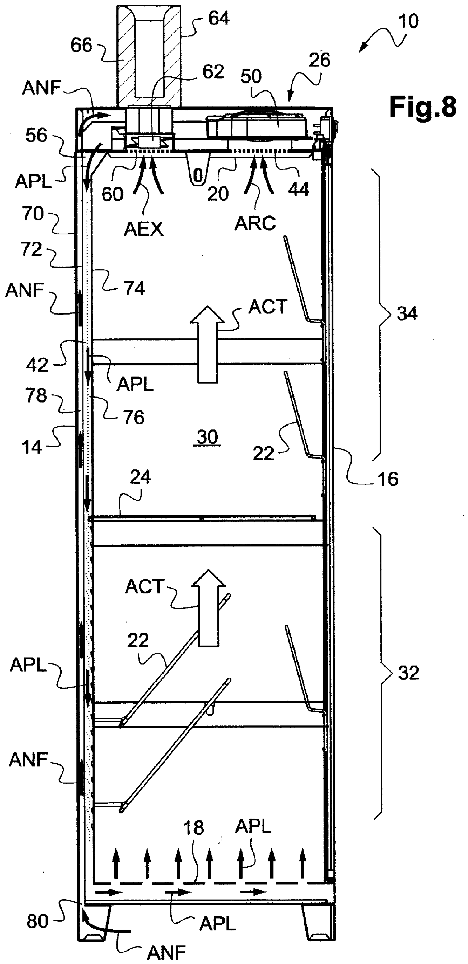

Pour la circulation des différents flux d'air, l'enceinte comprend :
des ouïes d'admission d'air neuf (flux d'air désigné ANF ci-après), qui sont des orifices d'entrée tels que les ouïes 36 en façade du bloc technique 26 et/ou les ouïes 38 ménagées en bas de la porte 16 et/ou les ouïes 80 d'admission d'air par le dessous de l'armoire (comme illustré sur la Figure 8) ;
des ouïes de diffusion d'air pulsé (flux d'air désigné APL ci-après), qui sont des orifices de sortie tels que les ouïes 40 situées en partie inférieure de la paroi de fond 14 et/ou les ouïes 68 ménagées dans la paroi de plancher 18 (comme illustré sur les Figures 7 et 8) ;
des ouïes d'aspiration 44 d'air recyclé (flux d'air désigné ARC ci-après), qui sont des orifices d'entrée de l'air chargé de contaminants tels que les ouïes 44 dans la paroi de plafond 20 (Figures 4 et 5) ; et des ouïes d'extraction de l'air contaminé à extraire (flux d'air désigné AEX ci-après), qui sont des orifices d'entrée de l'air chargé de contaminants tels que les ouïes 66 ménagées dans la paroi de plafond 20 (Figures 4 et 5).

De façon caractéristique de l'invention, les ouïes de diffusion 40, qui délivrent l'air ozoné et réchauffé produit par le bloc technique 26, sont disposées seulement (ou, à tout le moins, majoritairement) sur la partie inférieure de l'enceinte 30.

On va maintenant décrire en détail la structure du bloc technique 26, en référence aux Figures 3 à 5.

Pour amener l'air pulsé APL du bloc technique 26, situé au-dessus de l'enceinte 30, jusqu'aux ouïes de diffusion 40 situées en partie inférieure 32 de cette même enceinte, il est prévu un ou plusieurs collecteurs 42 de distribution de l'air pulsé APL, s'étendant par exemple dans la paroi de fond sur toute la hauteur de la partie supérieure 34 de l'enceinte 30.

Le bloc technique 26 comprend un motoventilateur 50 avec sa volute, qui aspire l'air neuf ANF par l'ouïe d'admission 36 pour le pulser vers un élément chauffant 52 puis vers un ou plusieurs générateurs d'ozone 54, de manière à délivrer sous pression un air pulsé chauffé et ozoné APL à l'entrée 56 de chacun des collecteurs verticaux 42, qui vont guider cet air pulsé APL jusqu'aux ouïes de diffusion 40 en partie inférieure 32 de l'enceinte 30.

Le motoventilateur 50 aspire également, via les ouïes d'aspiration 44, de l'air contaminé provenant du volume intérieur de l'enceinte 30, afin de le recycler vers les ouïes de diffusion 40 via les collecteurs 42.

La proportion, dans le flux d'air pulsé par le motoventilateur 50, de l'air recyclé ARC par rapport à l'air neuf ANF est déterminée par construction, en fonction des sections relatives des ouïes d'admission 36 (en façade du bloc technique 26) et 38 (dans la porte 16) et des ouïes d'aspiration 44 (dans la paroi de plafond 20, sous le bloc technique 26).

Le recyclage assure un brassage continu de l'air ozoné chauffé, ce qui accélère à la fois les mécanismes réactionnels de destruction des HAP et/ou des microorganismes et éventuellement le séchage des effets placés dans l'armoire, et donc réduit le temps total nécessaire à une décontamination complète.

Un autre avantage du recyclage de l'air est de pouvoir réutiliser une grande partie de l'air déjà chauffé, non saturé en humidité, pour accélérer le séchage des effets mouillés et donc de réduire la consommation globale d'énergie requise pour le processus.

Par ailleurs, une cassette amovible 58 peut être éventuellement montée sur un ou plusieurs des ouïes d'aspiration 44 pour diffuser avec l'air recyclé un produit désinfectant et/ou désodorisant qui viendra en contact avec les effets placés à l'intérieur de l'enceinte 30. Le produit est par exemple déposé sur un support absorbant qui est placé dans la cassette 58 pour permettre sa diffusion dans le volume de l'enceinte 30. Le principe de recirculation permet de maintenir plus longtemps l'efficacité du produit à l'intérieur de l'armoire.

Le bloc technique 26 comprend d'autre part un motoventilateur d'extraction 62 relié, en entrée, à des ouïes 60 d'extraction d'air AEX situées sur la paroi de plafond 20 et, en sortie, à une cheminée 64 d'évacuation vers l'atmosphère extérieure, cette cheminée incorporant un filtre 66, notamment un filtre à charbon actif. Ce filtre assure la rétention des particules contaminées après leur oxydation par l'ozone ainsi que la destruction du surplus d'ozone avant rejet à l'atmosphère, pour que la teneur finale en ozone de l'air rejeté à l'extérieur ne dépasse pas les seuils règlementaires autorisés, typiquement pas plus de 0,1 ppm.

La proportion, dans le flux d'air contaminé ACT, de l'air extrait AEX aspiré par le motoventilateur d'extraction 62 par rapport à l'air recyclé ARC aspiré par le motoventilateur principal 50 est déterminée par construction, en fonction des sections relatives des ouïes d'extraction 60 et des ouïes d'aspiration 44 ménagées dans la paroi de plafond 20 de l'enceinte.

La Figure 6 illustre un exemple de la variation du taux d'ozone au cours du temps qui a été relevée à l'intérieur de l'enceinte 30 dans des conditions expérimentales où l'ozone est produit en continu par un générateur d'ozone 54 unique, et où le motoventilateur d'extraction 62 est alimenté soit en continu (courbe A), soit de façon cyclique (courbe B) avec une alternance de cycles sans extraction de 15 minutes (procurant une élévation rapide du taux d'ozone) et de cycles d'extraction de 5 minutes destinés à assurer l'évacuation des produits de réaction (et engendrant une baisse corrélative du taux d'ozone dans l'enceinte).

On va maintenant décrire en référence à la Figure 7 la circulation des différents flux d'air au cours du traitement de décontamination avec le mode de réalisation de l'armoire 10 illustré aux Figures 1 à 5, dont on vient de décrire en détail la structure.

L'air neuf ANF aspiré depuis l'extérieur par le motoventilateur 50 via les ouïes d'admission 36 est mélangé à l'air recyclé ARC aspiré depuis l'intérieur de l'enceinte 30, puis réchauffé et rechargé en ozone par l'élément chauffant 52 et les générateurs d'ozone 54 pour produire un flux d'air pulsé sous pression APL ozoné et chauffé. On notera que de l'air neuf ANF peut également être admis depuis l'extérieur par les ouïes d'admission 38 formées dans la porte 32, du fait de la dépression créée dans l'enceinte 30 par l'aspiration d'air produite par le motoventilateur 50 via les ouïes d'aspiration 44.

L'air pulsé refoulé par le motoventilateur 50 est envoyé dans les collecteurs 42 sur toute la longueur où ceux-ci s'étendent dans la partie supérieure 34 de l'enceinte, depuis leur partie supérieure 56 au niveau du bloc technique 26 jusqu'aux diverses ouïes de diffusion 40 situées en partie inférieure 32 de l'enceinte.

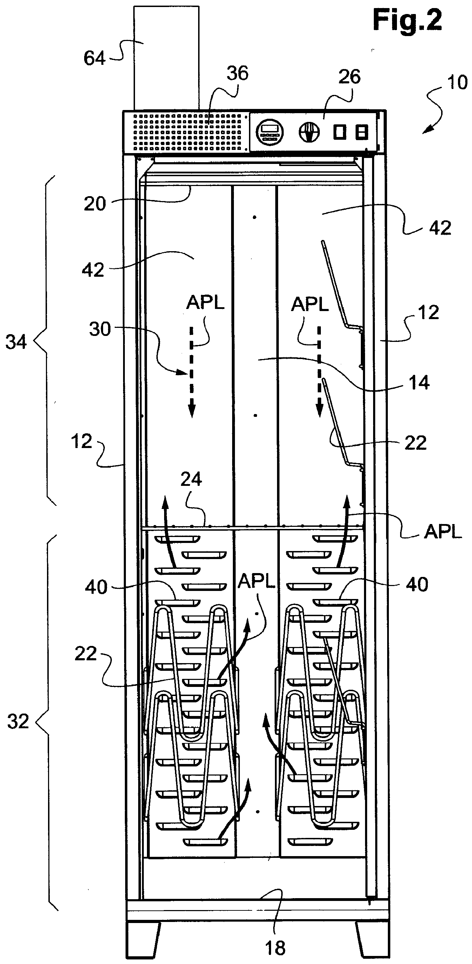

Ces ouïes de diffusion 40 peuvent, comme illustré notamment Figure 2, se répartir sur la paroi verticale de fond 14 de l'armoire mais également, en variante ou en complément, sur la paroi de plancher 18, comme illustré en 68 sur la Figure 7. Dans ce dernier cas, le plancher 18 est un plancher à double paroi et l'espace intercalaire entre ses deux parois est en communication avec l'extrémité inférieure du collecteur 42, pour permettre à l'air pulsé APL d'atteindre les ouïes de diffusion de plancher 68.

L'air pulsé APL délivré par les ouïes d'admission 40 et/ou 68 va alors circuler dans l'enceinte 30, sous le double effet de l'aspiration par les ouïes d'aspiration 44 situées à l'extrémité opposée, supérieure, de l'enceinte et de la convection naturelle de l'air chaud vers le haut. Au contact des effets à traiter, l'air pulsé va alors se charger en contaminants (air contaminé ACT sur les figures) et se refroidir progressivement. Si les effets à traiter sont encore mouillés, l'humidité extraite par le flux d'air chaud montant va, en sens inverse, descendre naturellement vers le bas de l'armoire où l'eau pourra se condenser, de sorte que l'air contaminé ACT atteignant le haut de l'enceinte avant d'être aspiré pour recirculation (flux d'air à recycler ARC) ne sera généralement pas saturé en humidité, celle-ci étant retombée vers les zones basses de l'enceinte.

On réalise ainsi à l'intérieur de l'enceinte un brassage continu de l'air, qui est particulièrement favorable au processus de décontamination pendant toute la durée de celui-ci, avec un minimum de turbulences qui seraient susceptibles de freiner le phénomène initial d'adsorption sans réaction exposée plus haut (mécanisme de Langmuir-Hinshelwood).

Par ailleurs, un flux de circulation d'air globalement laminaire, du bas vers le haut, permet de réduire les pertes thermiques avec les parois de l'armoire, ce qui est un avantage évident sur le plan de la consommation énergétique globale et donc du coût final de la décontamination.

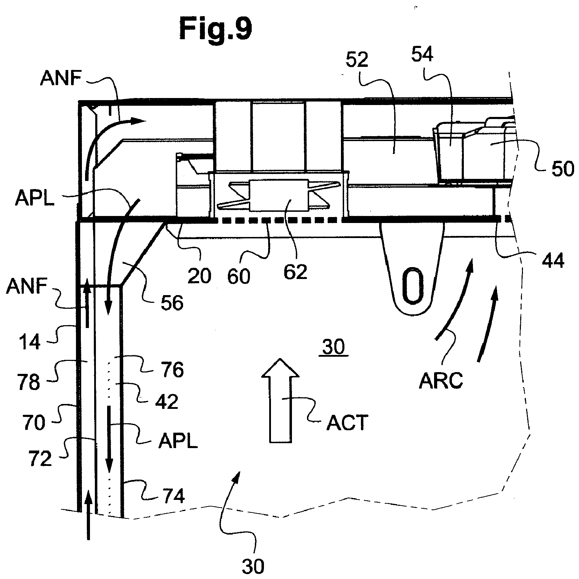

Les Figures 8 et 9 illustrent un second mode de réalisation de l'enceinte de l'invention, permettant d'améliorer encore plus le bilan thermique global du traitement de décontamination.

La structure et le fonctionnement de cette variante sont essentiellement les mêmes que celles du premier mode de réalisation décrit ci-dessus en référence aux Figures 1 à 5 et 7, et seuls les éléments qui diffèrent seront décrits.

La paroi verticale de fond 14 de l'armoire comprend ici une triple paroi 70, 72, 74, s'étendant en largeur au moins sur la largeur des collecteurs 42 et en hauteur de préférence sur toute l'étendue de l'enceinte comprise entre la paroi de plancher 18 et le bloc technique 26, au niveau de l'élément 56 recevant le flux air pulsé APL ozoné et réchauffé.

La triple paroi 70, 72, 74 définit deux gaines parallèles attenantes 76, 78 avec :
une gaine interne 76 du côté de l'intérieur de l'enceinte, et
une gaine externe 78 du côté de l'extérieur de l'armoire.

La gaine interne 76 constitue une conduite de liaison de l'unité de traitement d'air aux ouïes de diffusion 40, véhiculant vers le bas le flux air pulsé APL.

La gaine externe 78 peut, dans une première variante, constituer une couche statique d'isolation thermique (simple lame d'air statique ou garnissage d'un matériau isolant) interposée entre la gaine interne 76 véhiculant l'air chaud pulsé APL et l'environnement extérieur de l'enceinte, qui est à température ambiante. On limite ainsi fortement les pertes thermiques le long des collecteurs d'air pulsé 42 dans toute la région où l'air chaud ozoné, dont la température peut atteindre 40 ou 50 °C, voire 60 °C, est conduit sans être diffusé.

Dans une seconde variante, préférentielle, correspondant aux illustrations des Figures 8 et 9, la gaine externe 78 forme une conduite véhiculant vers le haut jusqu'à l'unité de traitement d'air 26 un flux d'air neuf ANF aspiré depuis le dessous de l'armoire par des ouïes d'admission d'air 80 situées en partie basse de celle-ci. Les ouïes 80 d'admission de l'air neuf ANF sont par exemple, comme illustré, situées dans le socle de l'armoire 10, avantageusement en remplacement des ouïes d'admission de façade 36 et/ou des ouïes d'admission de porte 38 du mode de réalisation précédent dont le fonctionnement avait été décrit à la Figure 7.

Les deux gaines attenantes forment entre elles un échangeur thermique, où le flux montant d'air neuf froid ANF aspiré dans la gaine externe 78 depuis le dessous de l'armoire par les ouïes d'admission 80 est progressivement réchauffé, au travers de la paroi intermédiaire 72, par le flux descendant d'air pulsé chaud APL refoulé par le motoventilateur 50 dans la gaine interne 76 à destination des ouïes de diffusion 40 (qui, dans l'exemple illustré Figure 8, sont des ouïes formées dans le plancher 18 de l'enceinte).

## Revendications

1. Une armoire de décontamination (10) appropriée à la dégradation des hydrocarbures aromatiques polycycliques et/ou à la destruction de microorganismes présents sur des effets contaminés, comprenant :
une enceinte close (30) s'étendant en hauteur et destinée à recevoir des effets à décontaminer, définie par des parois verticales (12, 12, 14, 16) incluant une porte (16), et par une paroi de plancher (18) et une paroi de plafond (20) de l'armoire ;
des moyens d'admission d'air neuf (36, 38, 80), communiquant avec l'extérieur de l'armoire et avec le volume intérieur de l'enceinte (30) ; et
une unité de traitement d'air (50, 52, 54), comprenant un premier motoventilateur (50) en communication amont et aval avec le volume intérieur de l'enceinte et avec au moins un générateur d'ozone (54) et
au moins un élément chauffant (52), de manière à créer à l'intérieur de l'enceinte un flux d'air pulsé (APL) venant en contact avec les
effets contaminés,
***caractérisée en ce que*** :
l'air pulsé (APL) est ozoné par l'unité de traitement d'air (50, 52, 54) à
une teneur d'ozone comprise entre 0,5 et 3 ppm,
***en ce que** :*
l'enceinte close définissant en hauteur une partie supérieure (34) et une partie inférieure (32), l'une au moins des parois verticales de l'armoire et/ou la paroi de plancher de l'armoire comprend des ouïes de diffusion (40) diffusant l'air pulsé (APL) et situées au niveau de ladite partie inférieure (32) de l'enceinte ; et
l'armoire comprend au moins un collecteur d'air pulsé (30), s'étendant le long de ladite partie supérieure de l'enceinte, et reliant l'unité de traitement d'air (50, 52, 54) aux ouïes de diffusion (40), de manière à recevoir le flux d'air pulsé (APL) produit par l'unité de traitement d'air pour le diffuser au niveau de ladite partie inférieure (32) de l'enceinte par les ouïes de diffusion (40) et produire dans l'enceinte un flux de circulation d'air globalement laminaire, du bas vers le haut,
*et **en ce que** :*
l'armoire comprend en outre une unité d'extraction (62, 64, 66) avec un second motoventilateur (62) relié en entrée à des ouïes d'extraction d'air (60) situées sur la paroi de plafond (20) de l'armoire et, en sortie, à une cheminée (64) d'évacuation vers l'atmosphère extérieure,
l'unité d'extraction (62, 64, 66) étant apte à i) extraire de l'enceinte une fraction (AEX) de l'air contaminé (ACT) chargé d'ozone et de particules, ii) filtrer cette fraction (AEX) de l'air contaminé (ACT) extraite, et iii) rejeter cette fraction d'air filtré à l'extérieur de l'armoire.

2. L'armoire de décontamination de la revendication 1, dans laquelle les moyens d'admission d'air neuf comprennent des ouïes d'admission (36, 38, 80) situées sur une paroi verticale (14) de l'armoire en partie inférieure de l'enceinte, et/ou dans la paroi de plancher (18) de l'armoire.

3. L'armoire de décontamination de la revendication 1, comprenant en outre dans la paroi de plafond (20) et/ou à proximité de la paroi de plafond (20) des ouïes d'aspiration (44) situées en amont du motoventilateur, pour la recirculation (ARC) de l'air contaminé (ACT), notamment d'air chaud non saturé en humidité.

4. L'armoire de décontamination de la revendication 3, dans lequel l'une au moins des ouïes d'aspiration (44) est munie d'une cassette désinfectante et/ou désodorisante (58).

5. L'armoire de décontamination de la revendication 1, dans laquelle l'une au moins des parois verticales (14) de l'armoire forme une paroi du collecteur d'air pulsé (42).

6. L'armoire de décontamination de la revendication 5, dans laquelle le collecteur d'air pulsé (42) comprend une triple paroi (70, 72, 74) définissant deux gaines (76, 78) s'étendant parallèlement sur au moins une partie du collecteur, avec :
du côté de l'intérieur de l'enceinte, une gaine interne (76) formant conduite de liaison de l'unité de traitement d'air (50, 52, 54) aux ouïes de diffusion (40) et véhiculant vers le bas le flux d'air pulsé (APL), et
du côté de l'extérieur de l'armoire, une gaine externe (78) formant couche statique d'isolation thermique.

7. L'armoire de décontamination de la revendication 5, dans laquelle le collecteur d'air pulsé (42) comprend une triple paroi (70, 72, 74) définissant deux gaines (76, 78) s'étendant parallèlement sur au moins une partie du collecteur, avec :
du côté de l'intérieur de l'enceinte, une gaine interne (76) formant une conduite reliant l'unité de traitement d'air (50, 52, 54) aux ouïes de diffusion (40) et véhiculant vers le bas le flux d'air pulsé (APL), et
du côté de l'extérieur de l'armoire, une gaine externe (78) formant une conduite reliant des ouïes d'admission d'air neuf (80) à l'unité de traitement d'air (50, 52, 54) et véhiculant vers le haut un flux d'air neuf (ANF),
les deux gaines attenantes formant échangeur thermique entre le flux d'air pulsé (APL) réchauffé sortant de l'unité de traitement d'air (50, 52, 54) et le flux d'air neuf (ANF) entrant dans l'unité de traitement d'air (50, 52, 54).

8. L'armoire de décontamination de la revendication 1, dans laquelle l'unité d'extraction et de filtrage comprend en outre :
un filtre actif (66),
et dans laquelle le second motoventilateur (62) est en communication avec le volume intérieur de l'enceinte (30) et avec le filtre actif (66) pour amener vers le filtre actif, avant rejet à l'extérieur de l'armoire par la cheminée, ladite fraction d'air contaminé chargé d'ozone (ACT).

9. L'armoire de décontamination de la revendication 1, dans laquelle la partie inférieure (32) de l'enceinte (30) recevant le flux d'air pulsé (APL) produit par l'unité de traitement d'air (50, 52, 54) s'étend sur 30 % à 70 %, de préférence 40 % à 60 %, de la hauteur totale du volume intérieur de l'enceinte (30).
